Europäisches Patentamt

European Patent Office

Office européen des brevets

(11) Veröffentlichungsnummer: **0 024 073**
**B1**

(12) **EUROPÄISCHE PATENTSCHRIFT**

(45) Veröffentlichungstag der Patentschrift:
**21.08.85**

(21) Anmeldenummer: **80900341.1**

(22) Anmeldetag: **10.01.80**

(86) Internationale Anmeldenummer:
**PCT/DE 80/00001**

(87) Internationale Veröffentlichungsnummer:
**WO 80/01751 (04.09.80 Gazette 80/20)**

(51) Int. Cl.⁴: **A 61 B 3/00,** G 12 B 5/00,
A 61 B 3/10, B 25 J 13/02

(54) **VORRICHTUNG ZUR FEINEINSTELLUNG EINES AUF EINER BASIS ANGEORDNETEN INSTRUMENTES IN ALLEN DREI RAUMRICHTUNGEN.**

(30) Priorität: **23.02.79 DE 2907099**

(43) Veröffentlichungstag der Anmeldung:
**25.02.81 Patentblatt 81/8**

(45) Bekanntmachung des Hinweises auf die Patenterteilung:
**21.08.85 Patentblatt 85/34**

(84) Benannte Vertragsstaaten:
**CH FR GB**

(56) Entgegenhaltungen:
**CH - A - 392 924**
**DE - B - 2 407 174**
**DE - C - 710 101**
**DE - C - 936 361**
**DE - C - 1 263 347**
**FR - A - 1 600 756**
**US - A - 2 940 357**
**US - A - 4 165 924**

(73) Patentinhaber: **Firma Carl Zeiss, Postfach 1369/1380, D-7082 Oberkochen (DE)**

(72) Erfinder: **KÖHLER, Kurt, Wiesenstrasse 2, D-7923 Königsbronn (DE)**

**Beschreibung**

Die vorliegende Erfindung betrifft eine Vorrichtung zur Feineinstellung eines auf einer Basis angeordneten Instrumentes in allen drei Raumrichtungen mittels eines zur Horizontaleinstellung allseitig schwenkbaren und zur Vertikaleinstellung drehbaren Betätigungsgliedes.

Aus CH-A-392 924 ist eine solche Vorrichtung bekannt, bei welcher das Betätigungsglied aus einer zur Vertikalverstellung des Instrumentes drehbaren Achse besteht, die von einem hohlen Handgriff umgeben ist. Dieser Griff ist direkt mit einer Kugel verbunden die allseitig schwenkbar in der Instrumentenbasis gelagert ist und mit der sich diese Basis auf einer Unterlage abstützt. Beim Verschwenken des Handgriffs rollt die Kugel auf der Unterlage ab und bewirkt dadurch eine Horizontalverschiebung der Basis. Die im Griff angeordnete Achse wird über einen, zusammen mit dem Handgriff mit einer Hand erfaßbaren Drehknopf gedreht und überträgt diese Bewegung über ein in der Kugel angeordnetes Kreuzgelenk auf eine zur Vertikalstellung des Instrumentes dienende Spindel.

Diese bekannte Vorrichtung hat den Nachteil, daß in einer beliebigen Verschwenkstellung des Handgriffs eine unbeabsichtigte Verdrehung dieses Griffes eine Horizontalverschiebung der Basis bewirken würde. Eine solche unbeabsichtigte Verdrehung ist möglich und deshalb nicht auszuschließen, da die Kugel eine Ausnehmung aufweist, welche in jeder Verschwenkstellung des Handgriffs die Weiterleitung einer Drehung der Achse auf die Spindel zur Vertikalverstellung ermöglicht.

DE-B-2 407 174 beschreibt eine andere Lösung, bei welcher eine unbeabsichtigte Horizontalverschiebung der Basis vermieden ist. Diese Vorrichtung besteht aus einem Handgriff, der über ein Kreuzgelenk mit einem drehbaren, zur Höhenverstellung des Instrumentes dienenden Teil der Basis verbunden ist und der einen beim Verschwenken des Handgriffs um den durch das Kreuzgelenk definierten Drehpunkt ohne Spiel gekoppelten inneren Hebel enthält. Dieser innere Hebel dient über ein gesondertes Lager nur zur Horizontalverstellung der Basis gegenüber der Grundplatte. Das untere Ende dieses inneren Hebels greift in einen auf der Grundplatte aufliegenden Teil der Instrumentenbasis ein, so daß eine Drehung dieses Hebels keine unbeabsichtigte Horizontalverschiebung der Basis bewirkt. Diese Lösung hat den Nachteil, daß die Instrumentenbasis flächig auf dem auf der Grundplatte aufliegenden Teil aufliegt und zur Horizontalverstellung gegenüber diesem Teil verschoben werden muß. Da hierzu die Reibung zwischen zwei Flächen überwunden werden muß, sind die aufzuwendenden Kräfte lastabhängig und es ist ein vollständig ruckfreies Verschieben nur bei sehr exakt ausgebildetem Flächenlager möglich. Ein solches Lager ist teuer und gegen Verschmutzung anfällig.

Derselbe Nachteil tritt bei einer anderen bekannten Feineinstellvorrichtung nach DE-C-1 263 347 auf, bei welcher sowohl zur Horizontalals auch zur Vertikalverstellung nur ein einteiliger Hebel vorgesehen ist, der in einer einzigen Lagerstelle allseitig verschwenkbar, jedoch bezüglich seiner Drehung mit der Lagerstelle gekoppelt ist. Das untere Ende dieses Hebels greift zur Vermeidung unbeabsichtigter Horizontalverschiebungen bei Hebeldrehung notwendigerweise in einen auf der Grundplatte aufliegenden Teil ein, gegen den sich die Instrumentenbasis flächig abstützt. Damit treten die geschilderten Nachteile eines Flächenlagers auf.

Die Nachteile eines Flächenlagers sind bei einer anderen, aus FR-A-1 600 756 bekannten Lösung vermieden. Auch bei dieser Lösung ist ein Handgriff vorgesehen, der über ein Kreuzgelenk mit einem drehbaren, zur Höhenverstellung des Instrumentes dienenden Teil der Basis verbunden ist und der einen, beim Verschwenken des Handgriffs um den durch das Kreuzgelenk definierten Drehpunkt ohne Spiel mit dem Griff gekoppelten, über ein gesondertes Lager zur Horizontalverstellung der Basis gegenüber der Grundplatte dienenden inneren Hebel enthält. Das untere Ende dieses inneren Hebels ist als Kugelfuß ausgebildet und liegt direkt auf der Grundplatte auf. Der Radius des Kugelfußes entspricht dem Abstand zwischen dem Drehpunkt des Griffes und der Grundplatte. Der innere Hebel ist in einer fest mit der Instrumentenbasis verbundenen Hülse allseitig schwenkbar gelagert. Diese Hülse ist außerhalb der Ebene des Kardangelenks angeordnet. Dadurch ist der Schwenkbereich des Betätigungsgliedes begrenzt, da schon bei relativ kleinen Schwenkwinkeln der innere Hebel an der Innenfläche des hohl ausgebildeten Handgriffs zum Anschlag kommt.

Es ist nun die Aufgabe der vorliegenden Erfindung eine Vorrichtung zur Feineinstellung eines auf einer Basis angeordneten Instrumentes in allen drei Raumrichtungen mit einem Betätigungsglied zu schaffen, das eine von einer Verschmutzung und/oder Abnützung des Lagers weitgehend unabhängige, leichtgängige Horizontalverstellung über einen großen Schwenkbereich ermöglicht, die von der Vertikaleinstell-Bewegung des Betätigungsgliedes vollständig entkoppelt ist.

Diese Aufgabe wird, ausgehend von der aus FR-A-1 600 756 bekannten Ausbildung durch eine Vorrichtung nach den Merkmalen des Patentanspruchs 1 gelöst.

Eine weitere vorteilhafte Ausbildung der Vorrichtung nach der Erfindung ist Gegenstand des Patentanspruchs 2.

Bei der Vorrichtung nach der Erfindung rollt also beim Verschwenken des Betätigungsgriffes das untere Ende des inneren Hebels auf der Grundplatte ab und verschiebt dabei über sein Lager in der Instrumentenbasis diese horizontal. Diese Abrollbewegung ist unabhängig vom Gewicht des auf der Basis angeordneten Instrumentes und sehr weitgehend unabhängig von Verschmutzungen und/oder Abnützungen des Kugelfußes des inneren Hebels. Die Vertikalverstellung des Instrumentes wird durch die Drehung des Betätigungsgriffes um seine Achse über ein eigenes Lager bewirkt. Da der innere Hebel in der Instrumentenbasis verschwenkbar, jedoch nicht drehbar gelagert ist, nimmt er an einer solchen Drehung des Betätigungsgriffes nicht teil, so daß eine unbeabsichtigte Horizontalverstellung der Basis in jeder Verschwenkstellung des Handgriffs mit Sicherheit vermieden ist. Da die Lagerhülse für den inneren Hebel in der Ebene des Kreuzgelenks angeordnet ist, behält der innere Hebel seine Lage zum Mantel des Betätigungsgriffes bei jeder Schwenklage bei, so daß ein großer Schwenkbereich des Betätigungsgliedes erreicht ist.

Da ein Drehen des Handgriffs keine große Kraft erfordert, ist es, ebenso wie bei allen mit einem einteiligen Betätigungsglied ausgestatteten Einstellvorrichtungen nach dem Stand der Technik auch bei der neuen Lösung im Prinzip möglich, bei einer reinen Horizontalverstellung, d. h. beim Verschwenken des Handgriffs unbeabsichtigt eine Drehung zu bewirken und damit eine geringe Vertikalverschiebung des Instrumentes auszulösen. Um auch diese Fehlerquelle auszuschalten ist es zweckmäßig den Handgriff aus zwei, bezüglich der Verschwenkung gekoppelten, jedoch bezüglich der Drehung entkoppelten Teilen auszubilden, wobei der obere Teil des Handgriffs mit dem oberen Ende des inneren Hebels fest verbunden und der untere Teil als drehbarer Griff ausgebildet ist. Bei sehr genauen Einstellungen legt dann der Beobachter seine Hand von oben auf den Handgriff und umfaßt mit den Fingern derselben Hand den unteren Teil. Die Horizontaleinstellung erfolgt durch reines Verschwenken des Handgriffs, das bequem über dessen oberen Teil bewirkt wird. Zur Vertikalverstellung wird dann bewußt der untere Teil des Handgriffs gedreht.

Es soll ausdrücklich betont werden, daß mit einem so ausgebildeten Handgriff eine Einhandbedienung der Einstellvorrichtung möglich ist, wobei jede unbeabsichtigte Verschiebung in horizontaler oder vertikaler Richtung vermieden werden kann.

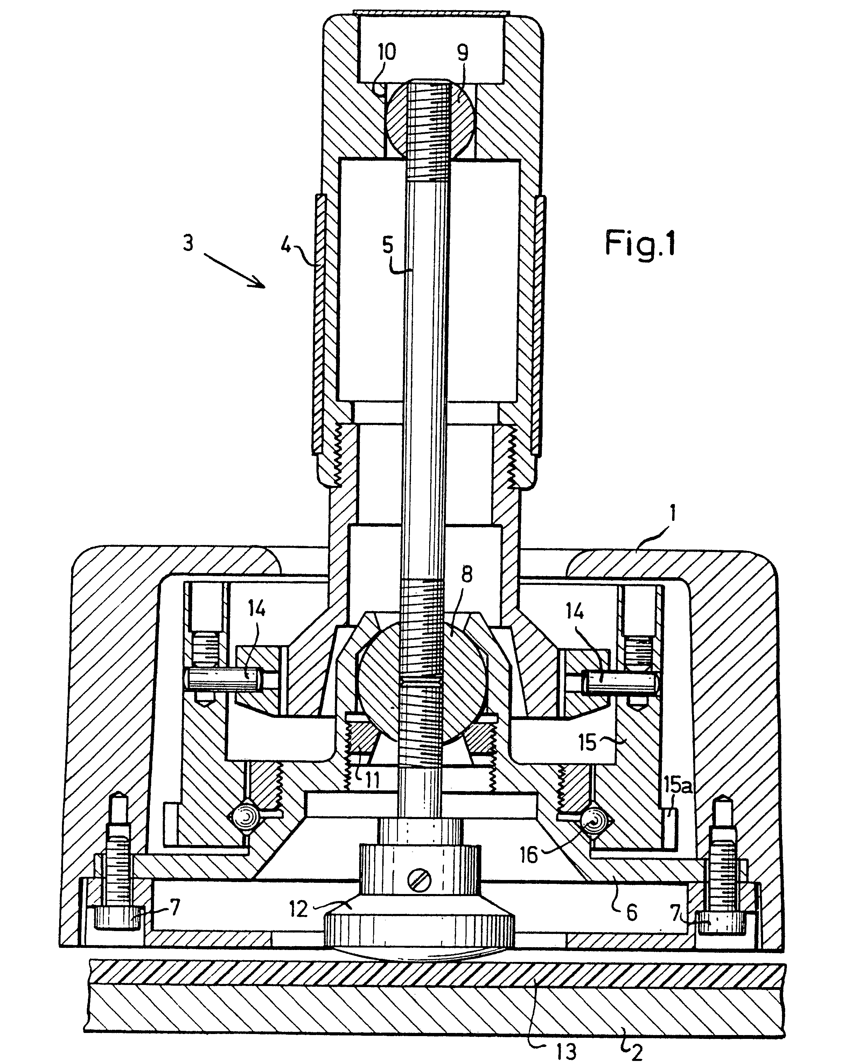

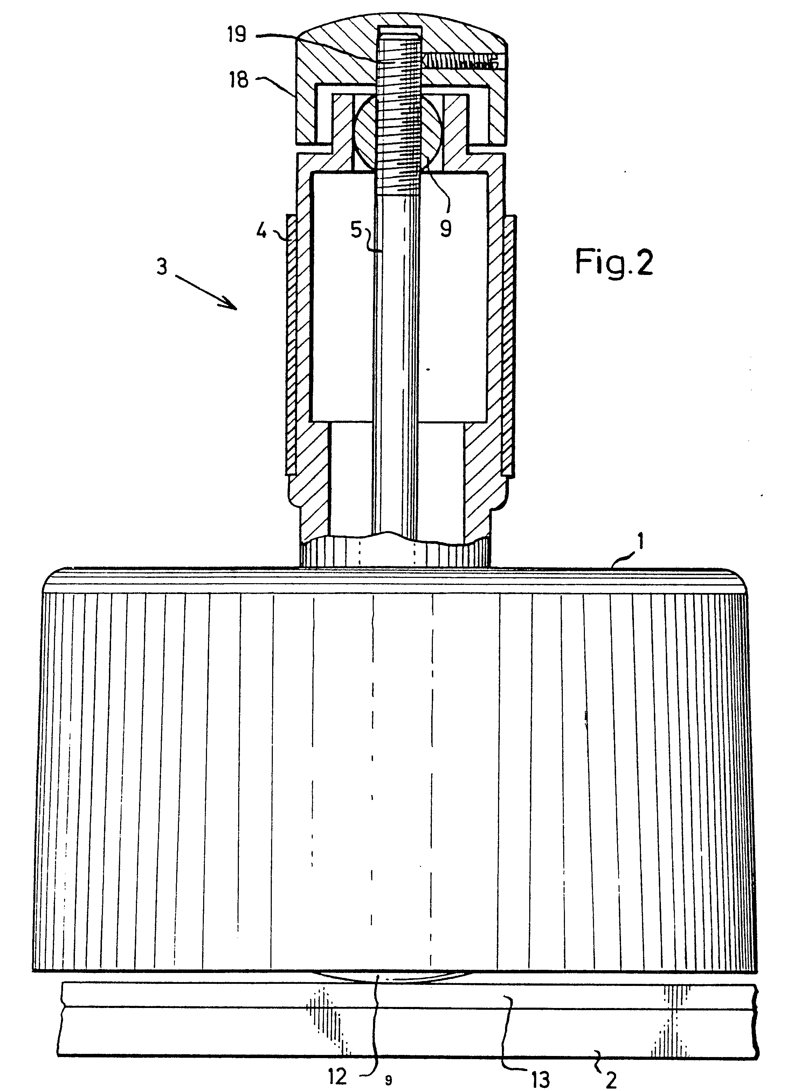

Die Erfindung wird im folgenden anhand der Fig. 1 und 2 der Zeichnungen näher erläutert. Im einzelnen zeigt

Fig. 1 einen Schnitt durch ein Ausführungsbeispiel einer gemäß der Erfindung ausgebildeten Instrumentenbasis in Vorderansicht;

Fig. 2 einen Schnitt durch ein Ausführungsbeispiel einer Instrumentenbasis mit zweiteiligem Betätigungsglied.

In Fig. 1 ist mit 1 eine Instrumentenbasis bezeichnet, welche auf einer Grundplatte 2 verschiebbar angeordnet ist. Die Basis 1 trägt ein Instrument, beispielsweise ein ophthalmologisches Gerät, das horizontal und vertikal zu verstellen ist. Zu einer solchen Einstellung in allen drei Raumrichtungen dient ein Betätigunsglied 3, das aus einem Handgriff 4 und einem in diesem angeordneten inneren Hebel 5 besteht.

In der Instrumentenbasis 1 ist eine Hülse 6 angeordnet und über Schrauben 7 fest mit der Basis 1 verbunden. Die Hülse 6 ist in ihrem oberen Teil als Lager ausgebildet, das eine fest mit dem inneren Hebel 5 verbundene kugelförmige Verdickung 8 aufnimmt. Der Hebel 5 trägt an seinem oberen Ende eine weitere kugelförmige Verdickung 9, welche in einer Hülse 10 des Handgriffs 4 gelagert ist. Das Teil 11 in der Hülse 6 ist so angezogen, daß der Hebel 5 mit seiner Verdickung 8 in der Hülse 6 allseitig verschwenkbar ist. Da die Reibung zwischen der Verdickung 8 und der Hülse 6 wesentlich höher eingestellt ist als die Reibung zwischen der oberen Verdickung 9 und der Hülse 10 dreht sich der Hebel 5 beim Drehen des Handgriffs 4 nicht mit.

Das untere Ende des Hebels 5 ist als Kugelfuß 12 ausgebildet, der direkt auf einem aus Kunststoff oder Gummi ausgebildeten Belag 13 der Grundplatte 2 aufliegt. Bei einer Verschwenkung des Hebels 5 rollt der Kugelkopf 12 auf der Unterlage 13 ab. Dabei wird über die Hülse 6 die Instrumentenbasis 1 gegenüber der Grundplatte 2 horizontal verschoben.

Der innere Hebel 5 ist vom Handgriff 4 umgeben, dessen unteres Ende über ein, schematisch mit 14 bezeichnetes Kreuzgelenk allseitig schwenkbar in einem zylindrischen Bauteil 15 gelagert ist. Teil 15 stützt sich über ein Kugellager 16 an der Hülse 6 ab und ist um diese Hülse drehbar. Eine solche Drehung wird durch Drehen des Handgriffes 4 bewirkt. Diese Drehung wird über das Kreuzgelenk 14 auf das Bauteil 15 übertragen. Ein mit diesem Teil 15 verbundener Zahnkranz 15a greift in weitere, hier nicht sichtbare Übertragungselemente ein, welche schließlich bei einer Drehung des Teiles 15 eine Vertikalverschiebung des Instrumentes auf der Basis 1 bewirken.

Zur Einstellung des Instrumentes ergreift also der Beobachter den Handgriff 4. Beim Verschwenken um den durch das Kreuzgelenk 14 definierten Drehpunkt rollt der Kugelfuß 12 auf der Unterlage 13 ab und verschiebt über die Lagerstelle des inneren Hebels 5 in der Hülse 6 die Instrumentenbasis horizontal. Beim Drehen des Handgriffs 4 bleibt der innere Hebel völlig unbeeinflußt. Über das Kreuzgelenk 14 wird vom Handgriff 4 das Bauteil 15 mitgedreht. Dadurch wird das Instrument vertikal verschoben.

Der Drehpunkt der Hebelverdickung 8 in der Hülse 6 füllt mit dem durch das Kreuzgelenk 14 definierten Drehpunkt des Betätigungsgliedes 3 zusammen. Die untere Fläche des Kugelfußes 12 ist sphärisch und ihr Radius entspricht dem Abstand zwischen dem Drehpunkt des Betätigungsgliedes 3 und der Unterlage 13.

Bei dem Ausführungsbeispiel der Fig. 1 ist die Horizontalverstellung von der Vertikalverstellung völlig entkoppelt. Es ist jedoch möglich, daß der Beobachter beim Verschwenken des Handgriffs 4 diesen unbeabsichtigt dreht und damit eine nicht gewünschte Vertikalverschiebung des Instrumentes bewirkt. Hier schafft das in Fig. 2 dargestellte Ausführungsbeispiel Abhilfe.

Bei diesem ist mit dem oberen Ende 19 des verlängerten inneren Hebels 5 ein Bauteil 18 fest verbunden. Dadurch ist das Bauteil 18 bezüglich der Verschwenkbewegung mit dem Handgriff 4 gekoppelt, bezüglich der Drehung des Griffes 4 von diesem jedoch entkoppelt.

Um eine sehr genaue Einstellung des Instrumentes zu bewirken, legt der Beobachter seine Hand von oben über das Betätigungsglied 3. Seine Hand ruht damit auf dem Teil 18 und die Finger umgreifen den Handgriff 4. Zur Horizontalverstellung wird jetzt lediglich über Teil 18 eine Verschwenkung des Betätigungsgliedes 3 bewirkt, wobei Handgriff 4 nicht unbeabsichtigt gedreht werden kann. Zur Vertikalverstellung wird der Handgriff 4 bewußt gedreht.

Es ist selbstverständlich möglich das hier schematisch dargestellte Betätigungsglied 3 im Bereich des drehbaren Handgriffs 4 speziell auszubilden um eine optimale Bedienung zu ermöglichen.

## Patentansprüche

1. Vorrichtung zur Feineinstellung eines auf einer Basis (1) angeordneten Instrumentes in allen drei Raumrichtungen mittels eines zur Horizontaleinstellung allseitig schwenkbaren und zur Vertikaleinstellung drehbaren Betätigunsgliedes (3), das aus einem Griff (4) besteht, der über ein Kreuzgelenk (14) mit einem drehbaren, zur Höhenverstellung des Instrumentes dienenden Teil (15) der Basis (1) verbunden ist und der einen, beim Verschwenken des Griffes (4) um den durch das Kreuzgelenk (14) definierten Drehpunkt ohne Spiel mit dem Griff (4) gekoppelten, über ein gesondertes Lager (6, 8) zur Horizontalverstellung der Basis (1) gegenüber einer Grundplatte (2, 13) dienenden inneren Hebel (5) enthält, dessen unteres Ende als Kugelfuß (12) ausgebildet ist und direkt auf der Grundplatte (13) aufliegt, dessen Radius dem Abstand zwischen dem Mittelpunkt einer kugelförmigen Verdikkung (8) im gesonderten Lager (6, 8) des Griffes (4) und der Grundplatte (2, 13) entspricht, und der mit dieser kugelförmigen Verdickung (8) allseitig schwenkbar in einer, fest mit der Instrumentenbasis (1) verbundenen Hülse (6) des gesonderten Lagers (6, 8) gelagert ist und zur Horizontalverstellung der Basis (1) gegenüber der Grundplatte (2, 13) dient, dadurch gekennzeichnet, daß die kugelförmige Verdickung (8) mit dem sie umfassenden Teil der Hülse (6) in der durch die Schwenkachsen des Kreuzgelenkes (14) aufgespannten Ebene angeordnet ist und daß der innere Hebel (5) verschwenkbar ist, sich jedoch beim Drehen des Handgriffs (4) nicht mitdreht.

2. Vorrichtung nach Anspruch 1, bei welcher das obere Ende des inneren Hebels (5) eine kugelförmige Verdickung (9) aufweist, welche in einer Hülse (10) des Griffes (3) gelagert ist, dadurch gekennzeichnet, daß die Reibung zwischen dem inneren Hebel (5) und der Hülse (6) in der Basis größer ist als diejenige zwischen dem inneren Hebel (5) und der Hülse (10) im Griff (3).

## Claims

1. A device for the fine adjustment in all three directions in space of an instrument arranged on a base (1) by means of an actuating member (3) which can be swung to all sides for horizontal adjustment and rotated for vertical adjustment, said actuating member consisting of a handle (4) connected via a universail joint (14) to a rotatable part (15) of the base (1) which effects vertical displacement of the instrument, said handle (4) further comprising an inner lever (5) which serves for the horizontal displacement of the base (1) with respect to a base plate (2, 13) via a separate bearing (6, 8), and which during swinging the handle (4) around the pivot defined by said universal joint (14) is coupled to this handle (4) without backlash, the lower end of said inner lever (5) being formed as a ball end (12) resting directly upon the base plate (13) and having a radius which corresponds to the distance between the center of a spherical thickening (8) arranged within said separate bearing (6, 8) and the base plate (2, 13), said inner lever (5) serving for the horizontal displacement of the base (1) with respect to the base plate (2, 13) and being supported pivotably in a sleeve (6) of said separate bearing (6, 8) which is firmly connected with the base (1), characterized in that said spherical thickening (8) together with that part of sleeve (6) embracing it is arranged in the plane being formed by the pivot axes of the universal joint (14), and that the inner lever (5) is swingable, but does not rotate when the handle (4) is rotated.

2. A device according to claim 1, in which the upper end of the inner lever (5) is provided with a spherical thickening (9), which is supported in a sleeve (10) of actuating member (3), characterized in that the friction between the inner lever (5) and the sleeve (6) in the base (1) is greater than the friction between the inner lever (5) and the sleeve (10) in the actuating member (3).

## Revendications

1. Dispositif pour le réglage précis d'un instrument disposé sur un support (1) suivant les trois directions dans l'espace, au moyen d'un élément de manoeuvre (3), que l'on peut incliner vers tous les côtés pour les réglage horizontal et que l'on peut tourner pour le réglage vertical, qui est formé d'une poignée (4) reliée par un joint de cardan (14) à une partie rotative (15) du support (1) servant au déplacement en hauteur de l'instrument et qui contient un levier intérieur (5), accouplé sans jeu avec la poignée (4) lors de l'inclinaison de celle-ci autour du point d'articulation défini par le joint de cardan (14) et servant par l'intermédiaire d'un palier particulier (6, 8) au déplacement horizontal du support (1) par rapport à une plaque semelle (2, 13), levier dont l'extrémité inférieure est réalisée comme un pied sphérique (12) appuyé directement sur la plaque semelle (13) et dont le rayon correspond à la distance entre le centre d'un épaississement sphérique (8) dans le palier particulier (6, 8) de la poignée (4) et la plaque semelle (2, 13), le levier, par cet épaississement sphérique (8), étant monté inclinable dans tous les sens dans une douille (6) du palier particulier (6, 8), qui sert au déplacement horizontal du support (1) par rapport à la plaque semelle (2, 13), caractérisé en ce que l'épaississement sphérique (8) avec la partie qui l'entoure de la douille (6) sont disposés dans le plan défini par les axes d'articulation du joint de cardan (14) et que le levier intérieur (5) est inclinable mais ne tourne pas avec la poignée (4) lorsque celle-ci est tournée.

2. Dispositif selon la revendication 1, où l'extrémité supérieure du levier intérieur (5) présente un épaississement sphérique (9) monté dans une douille (10) de la poignée (3), caractérisé en ce que le frottement entre le levier intérieur (5) et la douille (6) dans le support est plus grand que le frottement entre le levier intérieur (5) et la douille (10) dans la poignée (3).

0 024 073

Fig.1

0 024 073

Fig.2